# EUROPEAN PATENT APPLICATION

(11) **EP 0 659 743 A1**
(43) Date of publication of application: **28.06.1995**
(21) Application number: 93120915.9
(22) Date of filing: 27.12.1993
(51) Int. Cl.: C07D 211/42, C07D 211/30, A61K 31/445

(54) **Piperidine derivatives as inhibitors of platelet aggregation and their preparation**

(71) Applicant: HOECHST AKTIENGESELLSCHAFT, D-65929 Frankfurt am Main (DE)
(72) Inventor: The designation of the inventor has not yet been filed

(57) **Abstract**

Compounds of formula I 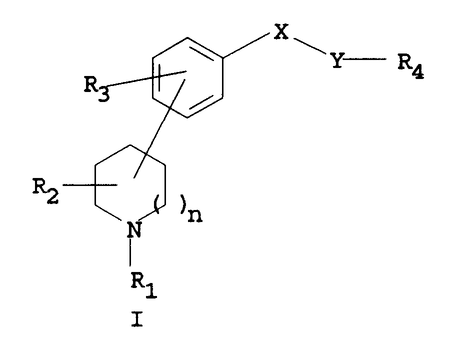
in which the substituents R₁-R₄, X,Y and n have the given meaning, show an activity as inhibitors of platelet aggregation.

## Description

The instant invention is directed to heterocyclic compounds, their preparation and their use, in particular their use as inhibitors of platelet aggregation.

Known inhibitors of platelet aggregation have, for example, the following formulas: 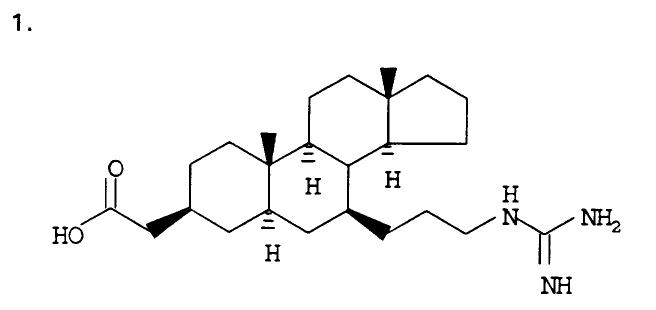
J. Amer. Chem. Soc., 1992, 114, 9699-9701. 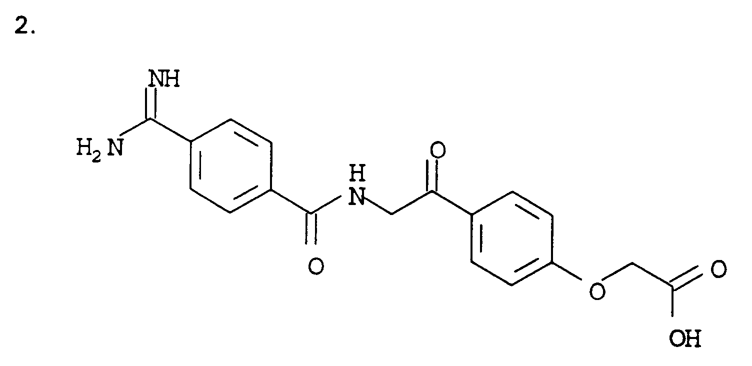
US Patent 5,039,805, 1991. 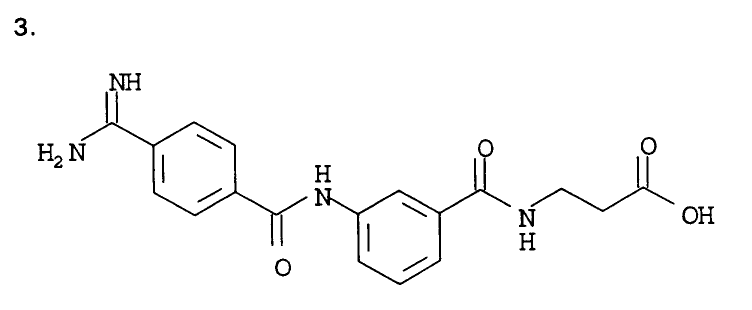
EP 0 483 667 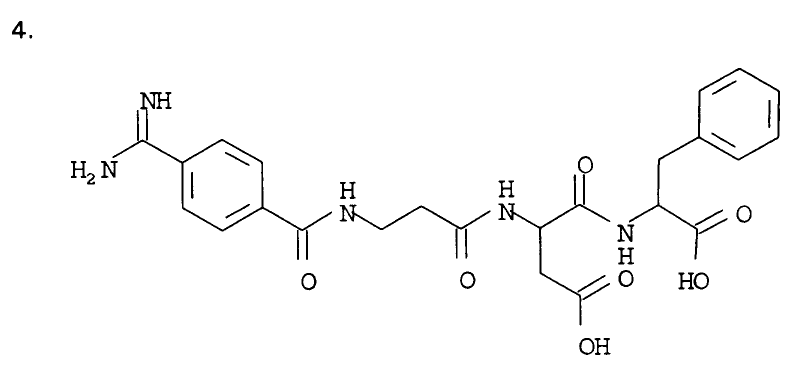
EP 0 445 796 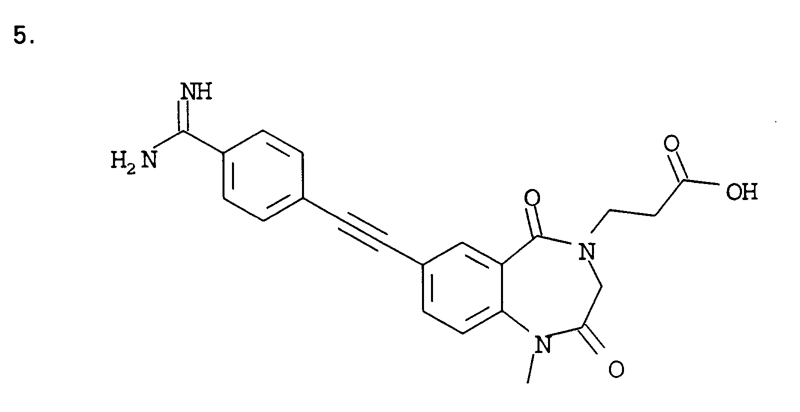
J. Med. Chem., 36 (21), 3039-3049, 1993. 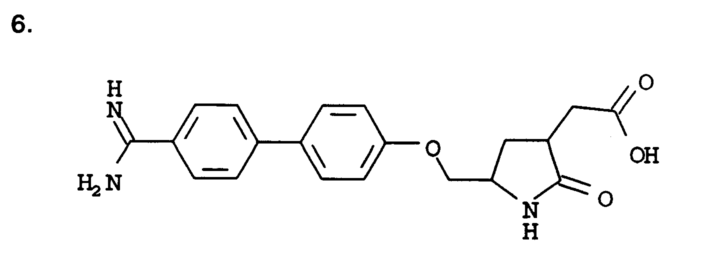
EP 0 483 667
Surprisingly, it has now been found, that the heterocyclic compounds according to the instant invention have an activity as inhibitors of platelet aggregation.

Accordingly, the subject of the instant invention are compounds of formula I 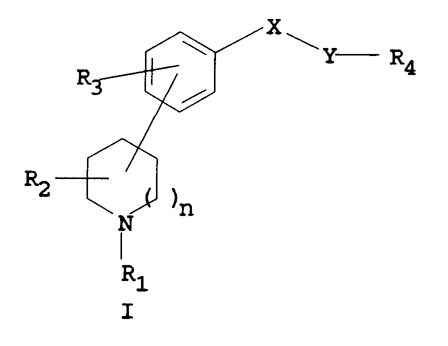
wherein,
- R₁ =: H, C₁-C₁₀-alkyl, C(=NH)-NH₂, C(=NH)-NHR₅ wherein R₅ is C₁-C₄-alkyl, C(=O)-C₁-C₄-alkyl, C(=O)-C₁-C₄-alkyl-aryl, C(=O)-aryl, C(=O)-substituted aryl, S(=O)₂-C₁-C₄-alkyl, S(=O)-aryl, S(=O)₂-aryl, S(=O)₂-substituted aryl;
C(=O)-C₁-C₁₀-alkyl-NH₂, C(=O)-C₁-C₁₀-alkyl-NH-C₁-C₄-alkyl, C(=O)-C₁-C₁₀-alkyl-N(C₁-C₄-alkyl)₂, C(=O)-C₁-C₁₀-alkyl-NH-C(=O)-NH₂, C(=O)-C₁-C₁₀-alkyl-NH-C(=O)-NH-C₁-C₄-alkyl, C(=O)-C₁-C₁₀-alkyl-NH-(=NH)-NH₂, C₁-C₄-alkyl-C(=NH)-NH₂, C(O)-C₁-C₄-alkyl-C(=NH)-NH₂, C(=O)-C₁-C₁₀-alkyl-NH-C(=NH)-NH-R₅, wherein R₅ is as defined above;
- R₂ =: H, one, two or three substituents, which are the same of different, selected from the group consisting of C₁-C₄-alkyl, OH, O-C₁-C₄-alkyl, O-C(=O)-C₁-C₄-alkyl, O-C(=O)-C₁-C₄-alkyl-aryl, O-C(=O)-NH₂, O-C(=O)-NH-C₁-C₄alkyl, O-C(=O)-NH-aryl, O-C(=O)-N(C₁-C₄-alkyl)₂;
- R₃ =: H, one, two or three substituents, which are the same of different, selected from the group consisting of C₁-C₄-alkyl, O-C₁-C₄-alkyl, F, Cl, Br, OH, O-C(=O)-C₁-C₄-alkyl, NH₂NH-C₁-C₄-alkyl, N(C₁-C₄-alkyl)₂, NH-C(=O)-C₁-C₄-alkyl, NH-C(=O)-aryl, NH-C(=O)-NH₂, COOH, C(=O)-O-C₁-C₄-alkyl, C(=O)-NH₂, C(=O)-NH-C₁-C₄-alkyl, C(=O)-N(C₁-C₄-alkyl)₂, C₁-C₄-COOH, C₁-C₄-alkyl-C(=O)-O-C₁-C₄-alkyl;
- X =: O, S, C(=O), C(=S), CH-C₁-C₄-alkyl, C(C₁-C₄-alkyl)₂, CHOH, ((OH)-C₁-C₄-alkyl;
- Y =: C₁-C₁₀-alkyl, C₂-C₁₀-alkenyl, C₂-C₁₀-alkynyl, C₁-C₄-alkyl-aryl, C₂-C₆-alkenyl-aryl, C₂-C₆-alkynyl-aryl, C₁-C₄-alkyl-substituted aryl, C₁-C₄-alkenyl-substituted-aryl, C₁-C₄-alkyl-heteroaryl, C₁-C₄-alkenyl-heteroaryl, aryl, substituted aryl, heteroaryl and substituted heteroaryl;
- R₄ =: H, one, two or three substituents, which are the same of different, selected from the group consisting of SO₃H, C(=O)-R₆, C₁-C₁₀-alkyl-C(=O)-R₆, C₂-C₁₀-alkenyl-C(=O)-R₆, C₂-C₁₀-alkynyl-C(=O)-R₆, O-C₁-C₁₀-alkyl-C(=O)-R₆, H-N-C(=O)-C₁-C₁₀-alkyl-C(=O)-R₆, N(C₁-C₄-alkyl)-C(=O)-C₁-C₁₀-alkyl-C(=O)-R₆, C(=O)-NH-C₁-C₁₀-alkyl-C(=O)-R₆; wherein R₆ is OH, C₁-C₄-alkyl, C₁-C₄-alkyl-aryl, aryl, substituted aryl, NH₂, NH-C₁-C₄-alkyl, N(C₁-C₄-alkyl)₂;
- n =: 0, 1 or 2
as well as the physiologically tolerable addition salts thereof.

The above-mentioned term alkyl means radicals such as CH₃, C₃H₇, C₄H₉ etc. which may also be branched e.g. (CH₃)₂CH.

The term alkenyl means an alkyl radical containing at least one double bond, e.g. -CH₂-CH = CH-CH₃ etc.

The term alkynyl refers to an alkyl radial containing at least one triple bond such as -CH₂-C≡C-CH₃.

The term aryl refers to cyclic planar unsaturated radicals such as phenyl, naphthyl etc.

The term heteroaryl refers to cyclic, planar unsaturated radicals containing hetero atoms like N, S, O etc. Examples of hetero aryl residues are pyridyl, quinyl, thienyl etc.

The term substituted aryl means aryl radicals substituted by atoms or groups of atoms such as C₁-C₄-alkyl, OH, COOH, C(=O)-O-C₁-C₄-alkyl, NH₂, O-C₁-C₄-alkyl, Cl, Br, F.

The term substituted heteroaryl means heteroaryl radicals substituted by atoms or groups of atoms such as C₁-C₄-alkyl, OH, O-C₁-C₄-alkyl, COOH, C(=O)-O-C₁-C₄-alkyl, NH₂, Cl, Br and F.

The physiologically tolerable addition salts of the compounds according to the instant invention are in particular salts with organic or inorganic acids, for example acetic acid, citric acid HCl or methylsulfonic acid.

Preferred are the compounds of the formula II 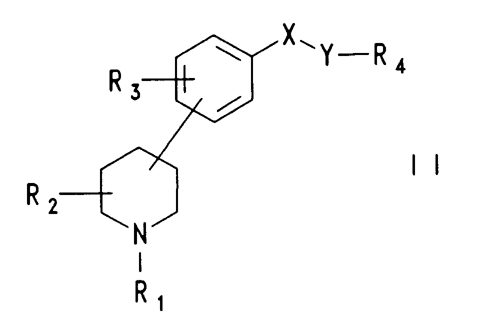
wherein,
- R₁ =: H, C₁-C₄-alkyl, C(=NH)-NH₂, C(=NH)-NHR₅ wherein R₅ is as defined above, C₁-C₄-alkyl-C(=NH)-NH₂, C(=O)-C₁-C₄-alkyl-C(=NH)-NH₂;
- R₂ =: H, one or two substituents, which are the same of different, selected from the group consisting of C₁-C₄-alkyl, O-C₁-C₄-alkyl, OH, O-C(=O)-C₁-C₄-alkyl, O-C(=O)-C₁-C₄-alkyl-aryl, O-C(=O)-NH-C₁-C₄-alkyl, O-C(=O)-NH-aryl;
- R₃ =: H, one or two substituents, which are the same of different, selected from the group consisting of OH, O-C₁-C₄-alkyl, O-C(=O)-C₁-C₄-alkyl, F, Cl, Br, NH₂, NH-C₁-C₄-alkyl, N(C₁-C₄-alkyl)₂, NH-C(=O)-C₁-C₄-alkyl, NH-C(=O)-aryl, COOH, C(=O)-O-C₁-C₄-alkyl;
- X =: C=O, CHOH, C(OH)-C₁-C₄-alkyl, C(C₁-C₄-alkyl)₂, CH(C₁-C₄-alkyl);
- Y =: C₁-C₄-alkyl, C₂-C₄-alkenyl, C₂-C₄-alkynyl, C₁-C₄-alkyl-aryl, C₂-C₄-alkenyl-aryl, C₂-C₄-alkynyl-aryl, C₁-C₄-alkyl-substituted aryl, C₁-C₄-alkenyl-substituted-aryl, C₁-C₄-alkyl-heteroaryl, C₁-C₄-alkenyl-heteroaryl, aryl, substituted aryl, heteroaryl and substituted heteroaryl;
- R₄ =: H, one or two substituents, which are the same of different, selected from the group consisting of SO₃H and groups represented by C(=O)-R₆, C₁-C₄-alkyl-C(=O)-R₆, C₂-C₄-alkenyl-C(=O)-R₆, C₂-C₄-alkynyl-C(=O)-R₆, O-C₁-C₄-alkyl-C(=O)-R₆, NH-C(=O)-C₁-C₄-alkyl-C(=O)-R₆, N(C₁-C₄-alkyl)-C(=O)-C₁-C₄-alkyl-C(=O)-R₆, C(=O)-NH-C₁-C₄-alkyl-C(=O)-R₆, wherein, R₆ is as defined above.

Particularly preferred are the compounds of formula III 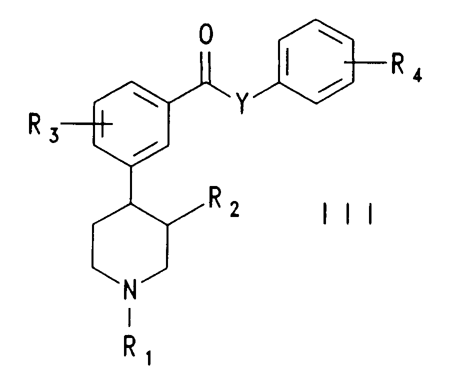
wherein,
- R₁ =: H, C₁-C₄-alkyl, C(=NH)-NH₂, C(=NH)-NHR₅ wherein R₅ is as defined above, C₁-C₄-alkyl-C(=NH)-NH₂, C(=O)-C₁-C₄-alkyl-C(=NH)-NH₂;
- R₂ =: H, C₁-C₄-alkyl, O-C₁-C₄-alkyl, OH, O-C(=O)-C₁-C₄-alkyl, O-C(=O)-C₁-C₄-alkyl-aryl, O-C(=O)-NH-C₁-C₄-alkyl, O-C(=O)-NH-aryl;
- R₃ =: H, or a substituent selected from the group consisting of OH, O-C₁-C₄-alkyl, O-C(=O)-C₁-C₄-alkyl, F, Cl, Br, NH₂, NH-C₁-C₄-alkyl, N(C₁-C₄-alkyl)₂, NH-C(=O)-C₁-C₄-alkyl, NH-C(=O)-aryl, COOH, C(=O)-O-C₁-C₄-alkyl;
- Y =: C₁-C₄-alkyl, C₂-C₄-alkenyl and C₂-C₄-alkynyl;
- R₄ =: H, or a substituent selected from the group consisting of SO₃H, and groups represented by C(=O)-R₆, C₁-C₄-alkyl-C(=O)-R₆, C₂-C₄-alkenyl-C(=O)-R₆, C₂-C₄-alkynyl-C(=O)-R₆, O-C₁-C₄-alkyl-C(=O)-R₆, NH-C(=O)-C₁-C₄-alkyl-C(=O)-R₆, N(C₁-C₄-alkyl)-C(=O)-C₁-C₄-alkyl-C(=O)-R₆, C(=O)-NH-C₁-C₄-alkyl-C(=O)-R₆, wherein, R₆ is OH, C₁-C₄-alkyl, C₁-C₄-alkyl-aryl, aryl, substituted aryl, NH₂, NH(C₁-C₄-alkyl), N(C₁-C₄-alkyl)₂.

The compounds of the present invention may contain asymmetric centers and therefore the invention encompasses all the stereoisomers in pure form or as mixtures.

Another subject of the instant invention is a process for the production of a compound as claimed in one or more of claims 1-3, wherein a compound formula V 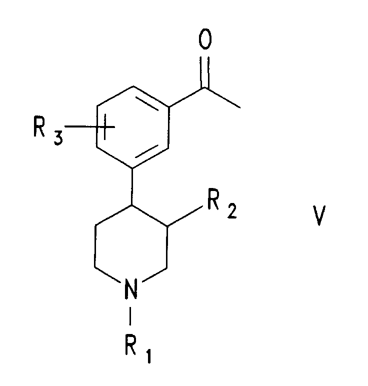
is reacted with a compound of formula VI to give a compound of formula VII 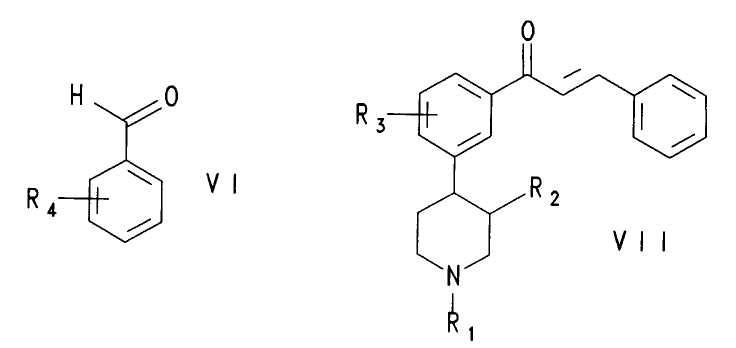
which in turn is optionally catalytically hydrogenated to a compound of formula VIII 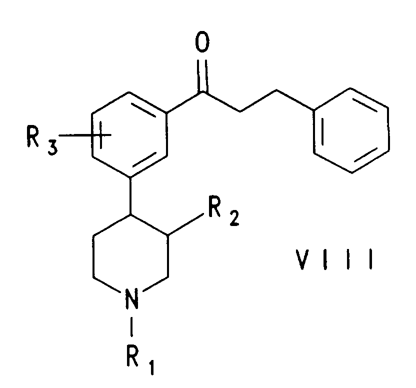
wherein the substituents have the denotations as outlined above.

The preparation of compounds of formula V are known in the literature (R. G. Naik at al.,Tetrahedron, 44, p 2081, 1988).

The compounds of the formula V when treated with the compounds of the formula VI preferably with bases such as sodium hydroxide 1-10 % in alcoholic solvents such as methyl alcohol give compounds of formula VII. Other bases such as sodium hydride in aprotic solvents such as dry N,N-dimethylformamide can also be used. The compounds of the formula VII can also be prepared by treating compounds of formula V with compounds of formula VI under acidic conditions such as mineral acids in alcoholic solvents or organic acids such as p-toluenesulfonic acids in non protic solvents such as benzene. The compounds of formula VIII are preferably prepared by catalytic hydrogenation of compounds of formula VII.

As already mentioned, the compounds according to the instant invention show an activity as inhibitors of platelet aggregation. Fibrinogen is a glycoprotein present as a normal component of blood plasma. It participates in platelet aggregation and fibrin formation in the blood clotting mechanism. When a blood vessel receives an injury the platelets binding to fibrinogen will initiate aggregation and form a thrombus. Interaction of fibrinogen with platelets occurs through a membrane glycoprotein complex gp. IIb/IIIa. Inhibitors of this interaction are useful in modulation of thrombus formation.

Another subject of the instant invention is the use of the compounds as described above as pharmaceuticals, as well as pharmaceuticals containing an amount of at least one of the compounds as described above, optionally together with physiologically tolerable carriers and/or excipients. The said pharmaceuticals are produced according to procedures known in the art.

Representative examples of preferred compounds of the present invention are given in Table 1 (refer to formula III, R₁ = Me, addition salts with HCl)

**Table 1**

| Compd. No. | R₃ | R₄ | R₂ | Y' | mp °C |
|---|---|---|---|---|---|
| 1 | 2,4,6-(OMe)₃ | p-O-CH₂-CO₂Me | OH | CH = CH | 145-146 |
| 2 | 2-OH, 4,6-(OMe)₂ | p-O-CH₂-CO₂Me | OH | CH = CH | 213-214 |
| 3 | 3-OH, 4,6-(OMe)₂ | p-O-CH₂CO₂Me | OH | CH₂CH₂ | 203-205 |
| 4 | 2-OH, 4,6-(OMe)₂ | p-O-CH₂CO₂H | OH | CH = CH | 233-234 |
| 5 | 2,4,6-(OMe)₂ | p-O-CH₂-CO₂H | OH | CH = CH | 236-237 |
| 6 | 2-OH, 4,6-(OMe)₂ | p-O-CH₂-CO₂Me | H | CH = CH | 217-218 |
| 7 | 2-OH, 4,6-(OMe)₂ | p-O-CH₂-CO₂Me | H | CH₂CH₂ | 194-195 |

Example: Preparation of compound 1 of table 1.

To a solution of the compound of formula V (R₁ = Me, R₁ = OH and R₃ = 2-OH, 4,6-(OMe)₂) (0.2 g) in 8 ml of 10 % NaOH solution in aqueous ethyl alcohol (1:1) is added a compound of formula VI (R₄ = O-CH₂-COOH) (0.223 g, 2 equiv.) and the mixture is stirred for 1 h. To the reaction mixture is added another equivalent of the aldehyde and stirred for one more hour. The reaction mixture is cooled in an ice bath and neutralized by addition of acetic acid to a pH of 7 to 7.5. The product is filtered and washed with minimum quantity of water and dried, to give compound 4, yield, 0.2 g, mp 233°-234°C. The compound 4 (0.5 g) is stirred in dry methyl alcohol with thionyl chloride (0.5 ml). After the addition is over the mixture is stirred at r.t. for one hour. The reaction mixture is worked up by pouring into water and basified by adding aqueous sodium carbonate and extracted with chloroform. The residue obtained after removal of chloroform is loaded on silica gel and eluted with 2 % MeOH in CHCl₃ + 1 % NH₄OH. The product is converted into the hydrochloride salt by adding ethereal HCl, mp 145-146°C, yield, 0.3 g.

The compounds of the above invention are tested for platelet binding inhibitory activity in the following assays:
1. In-vitro Human Platelet Aggregation in PRP:
   Blood is drawn from healthy human volunteers who had no medication for at least two weeks. 8.4 ml of venous blood are collected into 1.4 ml of ACD solution (citric acid 0.8 %, sodium citrate 2.2 %, hirudin 0.6 u/ml) and centrifuged for 10 min. at 120 g. The platelet rich plasma (PRP) is carefully removed and remaining blood is further centrifuged at 1200 g for 15 min. to separate platelet poor plasma (PPP).
   For aggregation studies the aggregometer is adjusted by using PPP and PRP. ADP is then added tp PRP which is kept at 37°C and stirred constantly at 1000 rpm. The aggregation of platelets causes an increase in transparency of PRP which is recorded.
2. Preparation of Gel Filtered Platelets (GFP):
   Platelet rich plasma (PRP) is collected as mentioned in the above procedure. Its pH is adjusted to 6.5 with ACD solution and then centrifuged at 285 g for 20 min.. The resulting pellet is resuspended in Tyrode's buffer (NaCl, 7.01 g/l; KCl, 0194 g/l; NaHCO₃, 1.01 g/l; Glucose, 1.09 g/l; NaH₂PO₄, 0.061 g/l; Hepes, 2.380 g/l; albumin, 3.5 g/l) and the platelet suspension is applied on to a ^{R}Sepharose CL 20 column. Equilibrium and elution is done with Tyrode's buffer. Platelets are recovered in the void volume. Final platelet concentration is adjusted to 4 X 108 platelets/ml.
   For the aggregation studies, GFP in Tyrodes buffer is mixed with calcium chloride (final concentration 0.5 µM) and an agonist (ADP or Thrombin) with or without test compound (1 to 300 µM) and incubated at 37°C for 3 min.. After the addition of fibrinogen (final conc. 1 mg/ml) the aggregation is recorded.

### Calculation of Results:

Aggregation maximum for PRP or GFP without any inhibitor is taken as 100 % aggregation and is compared with aggregation maximum of RPR or GFP incubated with the test compound.

The results are summarized below:

| Compound No. (see table 1) | IC₅₀(µM) Inhibitor of ADP induced Platelet aggregation |
|---|---|
| 1 | 151.0 (PRP) |
| | 11.5 (GFP) |
| 2 | 5.3 (PRP) |
| | 0.53 (GFP) |
| 3 | 21.0 (PRP) |
| | 1.8 (GFP) |
| 6 | 17.0 (PRP) |
| 7 | 8.0 (PRP) |
| | 2.0 (GFP) |

## Claims

1. Compounds of formula I 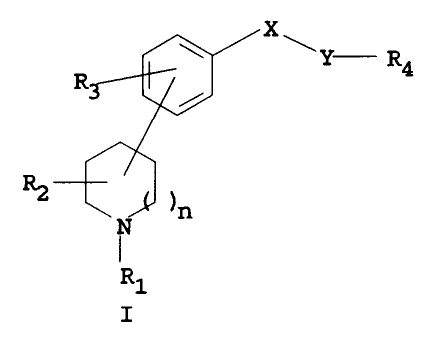 wherein,
R₁ = H, C₁-C₁₀-alkyl, C(=NH)-NH₂, C(=NH)-NHR₅ wherein R₅ is C₁-C₄-alkyl, C(=O)-C₁-C₄-alkyl, C(=O)-C₁-C₄-alkyl-aryl, C(=O)-aryl, C(=O)-substituted aryl, S(=O)₂-C₁-C₄-alkyl, S(=O)-aryl, S(=O)₂-aryl, S(=O)₂-substituted aryl;
C(=O)-C₁-C₁₀-alkyl-NH₂, C(=O)-C₁-C₁₀-alkyl-NH-C₁-C₄-alkyl, C(=O)-C₁-C₁₀-alkyl-N(C₁-C₄-alkyl)₂, C(=O)-C₁-C₁₀-alkyl-NH-C(=O)-NH₂, C(=O)-C₁-C₁₀-alkyl-NH-C(=O)-NH-C₁-C₄-alkyl, C(=O)-C₁-C₁₀-alkyl-NH-(=NH)-NH₂, C₁-C₄-alkyl-C(=NH)-NH₂, C(=O)-C₁-C₄-alkyl-C(=NH)-NH₂, C(=O)-C₁-C₁₀-alkyl-NH-C(=NH)-NH-R₅, wherein R₅ is as defined above;
R₂ = H, one, two or three substituents, which are the same of different, selected from the group consisting of C₁-C₄-alkyl, OH, O-C₁-C₄-alkyl, O-C(=O)-C₁-C₄-alkyl, O-C(=O)-C₁-C₄-alkyl-aryl, O-C(=O)-NH₂, O-C(=O)-NH-C₁-C₄-alkyl, O-C(=O)-NH-aryl, O-C(=O)-N(C₁-C₄-alkyl)₂;
R₃ = H, one, two or three substituents, which are the same of different, selected from the group consisting of C₁-C₄-alkyl, O-C₁-C₄-alkyl, F, Cl, Br, OH, O-C(=O)-C₁-C₄-alkyl, NH₂NH-C₁-C₄-alkyl, NH-C(=O)-C₁-C₄-alkyl, NH-C(=O)-aryl, N(C₁-C₄-alkyl)₂, NH-C(=O)-NH₂, COOH, C(=O)-O-C₁-C₄-alkyl, C(=O)-NH₂, C(=O)-NH-C₁-C₄-alkyl, C(=O)-N(C₁-C₄-alkyl)₂, C₁-C₄-COOH, C₁-C₄-alkyl-C(=O)-O-C₁-C₄-alkyl;
X = O, S, C(=O), C(=S), CH-C₁-C₄-alkyl, C(C₁-C₄-alkyl)₂, CHOH, C(OH)-C₁-C₄-alkyl;
Y = C₁-C₁₀-alkyl, C₂-C₁₀-alkenyl, C₂-C₁₀-alkynyl, C₁-C₄-alkyl-aryl, C₂-C₆-alkenyl-aryl, C₂-C₆-alkynyl-aryl, C₁-C₄-alkyl-substituted aryl, C₁-C₄-alkenyl-substituted-aryl, C₁-C₄-alkyl-heteroaryl, C₁-C₄-alkenyl-heteroaryl, aryl, substituted aryl, heteroaryl and substituted heteroaryl;
R₄ = H, one, two or three substituents, which are the same of different, selected from the group consisting of SO₃H, C(=O)-R₆, C₁-C₁₀-alkyl-C(=O)-R₆, C₂-C₁₀-alkenyl-C(=O)-R₆, C₂-C₁₀-alkynyl-C(=O)-R₆, O-C₁-C₁₀-alkyl-C(=O)-R₆, H-N-C(=O)-C₁-C₁₀-alkyl-C(=O)-R₆, N(C₁-C₄-alkyl)-C(=O)-C₁-C₁₀-alkyl-C(=O)-R₆, C(=O)-NH-C₁-C₁₀-alkyl-C(=O)-R₆; wherein R₆ is OH, C₁-C₄-alkyl, C₁-C₄-alkyl-aryl, aryl, substituted aryl, NH₂, NH-C₁-C₄-alkyl, N(C₁-C₄-alkyl)₂;
n = 0, 1 or 2,
as well as the physiologically tolerable addition salts thereof.

2. Compounds of formula II as claimed in claim 1, 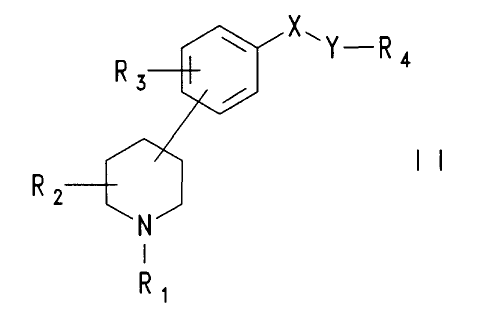 wherein
R₁ = H, C₁-C₄-alkyl, C(=NH)-NH₂, C(=NH)-NHR₅ wherein R₅ is as defined in claim 1 C₁-C₄-alkyl-C(=NH)-NH₂, C(=O)-C₁-C₄-alkyl-C(=NH)-NH₂;
R₂ = H, one or two substituents, which are the same of different, selected from the group consisting of C₁-C₄-alkyl, O-C₁-C₄-alkyl, OH, O-C(=O)-C₁-C₄-alkyl, O-C(=O)-C₁-C₄-alkyl-aryl, O-C(=O)-NH-C₁-C₄-alkyl, O-C(=O)-NH-aryl;
R₃ = H, one or two substituents, which are the same of different, selected from the group consisting of OH, O-C₁-C₄-alkyl, O-C(=O)-C₁-C₄-alkyl, F, Cl, Br, NH₂, NH-C₁-C₄-alkyl, N(C₁-C₄-alkyl)₂, NH-C(=O)-C₁-C₄-alkyl, NH-C(=O)-aryl, COOH, C(=O)-O-C₁-C₄-alkyl;
X = C = O, CHOH, C(OH)-C₁-C₄-alkyl, C(C₁-C₄-alkyl)₂, CH(C₁-C₄-alkyl);
Y = C₁-C₄-alkyl, C₂-C₄-alkenyl, C₂-C₄-alkynyl, C₁-C₄-alkyl-aryl, C₂-C₄-alkenyl-aryl, C₂-C₄-alkynyl-aryl, C₁-C₄-alkyl-substituted aryl, C₁-C₄-alkenyl-substituted-aryl, C₁-C₄-alkyl-heteroaryl, C₁-C₄-alkenyl-heteroaryl, aryl, substituted aryl, heteroaryl and substituted heteroaryl;
R₄ = H, one or two substituents, which are the same of different, selected from the group consisting of SO₃H and groups represented by C(=O)-R₆, C₁-C₄-alkyl-C(=O)-R₆, C₂-C₄-alkenyl-C(=O)-R₆, C₂-C₄-alkynyl-C(=O)-R₆, O-C₁-C₄-alkyl-C(=O)-R₆, NH-C(=O)-C₁-C₄-alkyl-C(=O)-R₆, N(C₁-C₄-alkyl)-C(=O)-C₁-C₄-alkyl-C(=O)-R₆, C(=O)-NH-C₁-C₄-alkyl-C(=O)-R₆, wherein, R₆ is as defined in claim 1.

3. Compounds of formula III, as claimed in claims 1 or 2 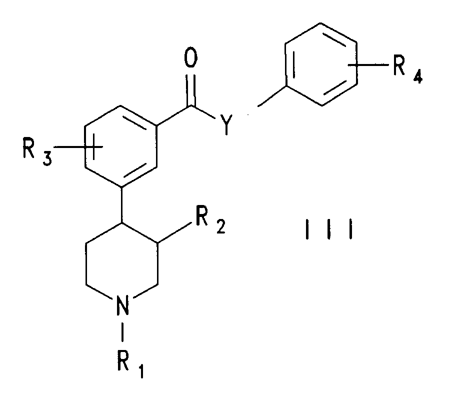 wherein,
R₁ = H, C₁-C₄-alkyl, C(=NH)-NH₂, C(=NH)-NHR₅ wherein R₅ is as defined in claim 1 C₁-C₄-alkyl-C(=NH)-NH₂, C(=O)-C₁-C₄-alkyl-C(=NH)-NH₂.
R₂ = H, C₁-C₄-alkyl, O-C₁-C₄-alkyl, OH, O-C(=O)-C₁-C₄-alkyl, O-C(=O)-C₁-C₄-alkyl-aryl, O-C(=O)-NH-C₁-C₄-alkyl, O-C(=O)-NH-aryl;
R₃ = H, or a substituent selected from the group consisting of OH, O-C₁-C₄-alkyl, O-C(=O)-₁-C₄-alkyl, F, Cl, Br, NH₂, NH-C₁-C₄-alkyl, N(C₁-C₄-alkyl)₂, NH-C(=O)-C₁-C₄-alkyl, NH-C(=O)-aryl, COOH, C(=O)-O-C₁-C₄-alkyl;
Y = C₁-C₄-alkyl, C₂-C₄-alkenyl and C₂-C₄-alkynyl:
R₄ = H, or a substituent selected from the group consisting of SO₃H, and groups represented by C(=O)-R₆, C₁-C₄-alkyl-C(=O)-R₆, C₂-C₄-alkenyl-C(=O)-R₆, C₂-C₄-alkynyl-C(=O)-R₆, O-C₁-C₄-alkyl-C(=O)-R₆, NH-C(=O)-C₁-C₄-alkyl-C(=O)-R₆, N(C₁-C₄-alkyl)-C(=O)-C₁-C₄-alkyl-C(=O)-R₆, C(=O)-NH-C₁-C₁₀-alkyl-C(=O)-R₆, wherein, R₆ is OH, C₁-C₄-alkyl, C₁-C₄-alkyl-aryl, aryl, substituted aryl, NH₂, NH(C₁-C₄-alkyl), N(C₁-C₄-alkly)₂.

4. A process for the production of a compound as claimed in one or more of claims 1-3, wherein a compound formula V 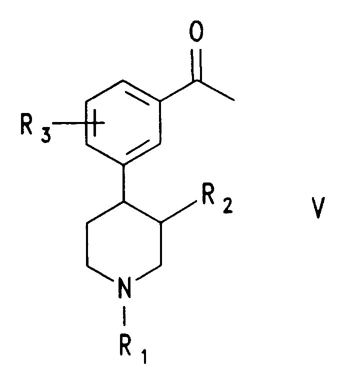 is reacted with a compound of formula VI to give a compound of formula VII 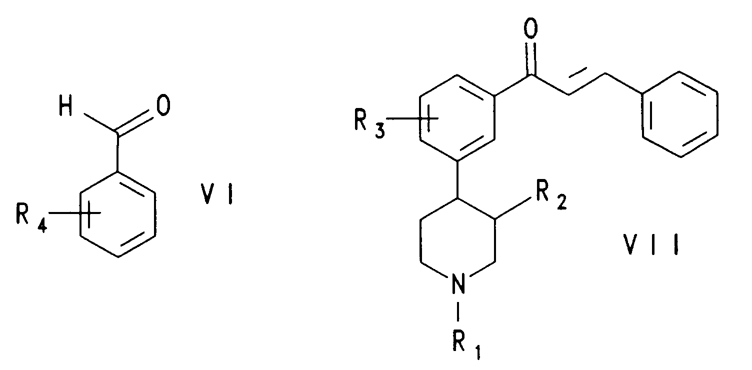 which in turn is optionally catalytically hydrogenated to a compound of formula VIII 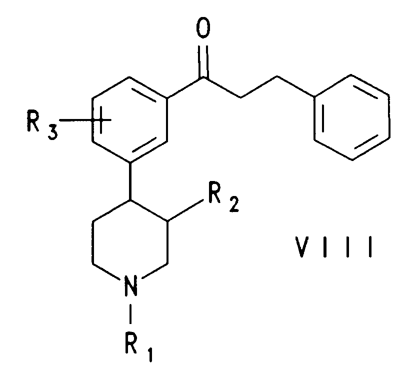 wherein the substituents R₁ - R₄ have the denotations.

5. The compounds as claimed in one or more of claims 1-3 as a pharmaceutical

6. The use of the compounds as claimed in one or more of claims 1-3 for the production of a pharmaceutic with an activity against platelet aggregation.

7. A pharmaceutical having an active amount of one or more compounds as claimed in one or more of claims 1-3 optionally together with physiologically tolerable carriers and/or excipients.

8. A process for the production of a pharmaceutical as claimed in claim 7, wherein at least one compound as claimed in claims 1-3 is brought, optionally together with physiologically tolerable carriers and/or excipients into a suitable form for administration.
